Europäisches Patentamt

European Patent Office

Office européen des brevets

(11) Publication number: **0 109 124**
A2

(12) **EUROPEAN PATENT APPLICATION**

(21) Application number: 83201585.3

(51) Int. Cl.³: **C 08 L 25/16,** C 12 N 5/00

(22) Date of filing: 04.11.83

(30) Priority: 11.11.82 NL 8204364

(43) Date of publication of application: 23.05.84
Bulletin 84/21

(84) Designated Contracting States: **BE DE FR GB IT NL SE**

(71) Applicant: **AKZO N.V., Velperweg 76, NL-6824 BM
Arnhem (NL)**

(72) Inventor: **Hoefs, Cornelis Adrianus Maria, de Savornin
Lohmanstraat 50, NL-6662 AK Elst (NL)**
Inventor: **Magre, Eduard Pieter, Hyacintenlaan 9,
NL-6866 DV Heelsum (NL)**

(74) Representative: **Sieders, René et al, P.O. Box 314,
NL-6800 AH Arnhem (NL)**

(54) Cell culture microcarriers based on the polymerization product of an aromatic monovinyl hydrocarbon.

(57) Cell culture microcarriers having a diameter in the range of from 60 to 300 μm, the carrier material substantially consisting of the polymerization product of cross-linked polyvinyl toluene provided with negatively or positively charges groups.

The microcarriers display a low degree of swelling and the adhesion of the cells in the inital stage of the breed is excellent.

Cell culture microcarriers based on the polymerization product of an aromatic monovinyl hydrocarbon

The invention relates to cell culture microcarriers having a diameter in the range of from 60 to 300 µm, the carrier material consisting of the polymerization product of an aromatic monovinyl hydrocarbon provided with negatively or positively charged groups, and to a method of growing anchorage-dependent cells in microcarrier culture with the aid of these cell culture microcarriers.

Cell culture microcarriers of the above-mentioned type are described, among other places, in two articles by Johansson and Nielsen in Develop. biol. Standard 46, pp. 125-129 and pp. 131-136 (S. Karger, Basel 1980).

The carrier material consists of polystyrene beads having a particle size in the range of 160 to 300 µm. The density of the beads is 1,05 $g/cm^3$. They carry 2 to $14 \times 10^{14}$ negatively charged groups per $cm^2$ of surface area.

Although these beads offer several advantages over commonly applied cell culture microcarriers based on polydextran in that they display a lower degree of swelling, have a narrow particle size distribution and good mechanical properties, cell attachment at the beginning of the cultivation process is not at all found to be satisfactory. One remedy recommended to that end is to refrain from stirring in the early stages of the cultivation process. As a result, however, the beads will precipitate, which also does not contribute to obtaining a satisfactory and uniform distribution of the cells over the beads.

The present invention provides cell culture carriers which do not display the above-mentioned disadvantages or only to a greatly reduced extent. The invention consists in that with cell culture carriers of the known type mentioned in the opening paragraph the polymerization product substantially consists of cross-linked polyvinyl toluene.

It is particularly surprising that upon using this polymer the above problems are hardly met if at all. It is of importance that the polyvinyl toluene should contain a cross-linking agent. It is preferred that use should be made of a cross-linking agent in an amount which is in the range of 0,1 to 7% by weight, calculated on the polyvinyl toluene.

0109124
ACR 1889 B

Use of a cross-linking agent in an amount of less than 0,1% by weight will cause the beads to swell unduly strongly and disintegrate in a solvent upon introducing ionic groups-containing substituents. Use of a cross-linking agent in an amount higher than 7% by weight is generally detrimental to the mechanical properties in that a relatively brittle polymer is obtained.

Preference is generally given to polyvinyl toluene beads containing a cross-linking agent in an amount of 0,1 to 4% by weight, preferably 0,5 to 2,5% by weight.

As cross-linking agents a variety of compounds may be employed. It is desirable that as such use should be made of polyvinyl compounds, preferably divinyl and trivinyl, aromatic, aliphatic and heterocyclic compounds. As examples may be mentioned divinyl toluene, divinyl xylene, divinyl naphthalene, divinyl pyridine, ethylene glycol dimethacrylate, ethylene glycol diacrylate, divinyl ethyl benzene, divinyl sulphone, divinyl ketone, divinyl sulphide, diallyl fumarate, diallyl maleate, acrylic esters such as divinyl acrylates, divinyl fumarates, divinyl oxalates and the corresponding trivinyl compounds such as trivinyl benzene, trivinyl citrate, trivinyl aconitate and trivinyl phosphates.

In actual practice preference is given to the relatively inexpensive divinyl benzene.

The polymerization product according to the invention may, in addition to a cross-linking agent, contain other vinyl monomers.

As a rule, the monovinyl monomers mixture may contain monomers other than vinyl toluene in a maximum amount of 10% by weight.

Examples of suitable monovinyl monomers are styrene, o-, m- and p-ethyl styrene, tert.butyl styrene, vinyl naphthalene, vinyl anthracene and homologous compounds. Of the vinyl toluene all three isomers, viz. o-, m- and p-vinyl toluene qualify for use in the preparation of the cell culture microcarriers according to the invention.

The preparation of the cell culture microcarriers according to the invention may be effected by various well-known prior art methods. The preparation may be carried out by block polymerization, but preference is given to emulsion polymerization or suspension polymerization. The polymerization is carried out in the presence of catalysts initiating the formation of free radicals. As examples may be mentioned azobisisobutyronitrile and oxidizing catalysts such as sodium persulphate or potassium persulphate, but preference is given to oxygen or organic peroxides such as benzoyl peroxide, lauryl peroxide, and tert.alkyl peroxide. It is preferred that the catalyst should be used in an amount ranging between about 0,5 to 5 parts per 100 parts of monomer.

Providing positively or negatively charged groups also may be effected by well-known prior art methods.

Positively charged groups may be provided for instance as indicated in United States Patent Specification 2 591 574. In the process described in it the polymr is first haloalkylated. This step involves introducing into the polymer a plurality of bromoalkyl or chloroalkyl groups, that is groups of the general formula $-C_nH_{2n}X$, wherein n is an integer of a value from one to four and X is a chlorine or bromine atom. As halo-alkylating agent may be used a mixture of an aldehyde and a halogen acid (e.g., paraformaldehyde and hydrochloric acid) or a dihalo alkane and a Friedel-Crafts catalyst (e.g., ethylene dichloride and aluminium chloride), or a haloether and a Friedel Crafts catalyst. An activated microcarrier also may be obtained by copolymerization of, say, p-chloromethyl styrene.

It is preferred that the resulting haloalkylated copolymer should subsequently be treated with a swelling agent (e.g., by washing with dioxane) and then reacted with a solution of a primary and/or secondary amine, resulting in an insoluble, cross-linked, polymeric amine salt, which may be converted into the free amine by washing with sodium hydroxide. Alternatively, use may be made of a tertiary amine, which results in obtaining a strongly basic polymeric amine. As solvent for the amine preferably an agent is chosen which also acts as a swelling agent for the polymer.

An example of a suitable solvent is dioxane.

The amines are used in the form of the free base. The amines which are preferred are those which are primary or secondary and in which the amino group or groups are attached to a hydrocarbon group. Optionally, said last-mentioned group may carry a substituent, provided that it does not interfere with the amination reaction of the haloalkylated polymer. Of course, also mixtures of various primary and/or secondary amines may be employed. The hydrocarbon portion of the amine may be aromatic, ali-phatic, cycloaliphatic, araliphatic, or alkyl aromatic. As examples of amines which are suitable in this invention may be mentioned:

methylamine, dimethylamine, n-butylamine, isobutylamine, dibutylamines, aniline, benzidine, o-, m-, and p-toluidines, xylidines, α- and ß-naphthylamine, naphthalene diamines, benzylamine, dibenzylamine, phenylene
diamine, benzyl aniline, benzyl ethylamine, methyl aniline, cyclohexylamine, dicyclohexylamine, diethylene triamine, triethylene tetramine,
tetraethylene pentamine, and propylene diamine.

An example of a tertiary amine is trimethylamine. Strongly basic polymers may be obtained by reacting a feebly basic polymer with methyl
iodide or methyl chloride or by reaction with a tertiary amine. A man
skilled in the art will have no difficulty in choosing such conditions
for a given polymer system of a particular particle size as will result
in optimum cell growth per unit of polymer weight.
It has been found that cell culture carriers having optimum properties
are generally obtained if the amount of positively charged groups per
unit of area is in the range of from 0,1 to 5 meq/g.

The cell culture carriers may be negatively charged in accordance with,
for instance, the method described in United States Patent Specification
2 500 149. In order to obtain products of as light as a colour as possible it is recommended that the sulphonation should be carried out in
the presence of phosphorus trichloride. In that case, too, the proportion of negatively charged groups to be provided depends on the given
polymer system and the particle size of the carrier material.
Favourable results are generally obtained using $2 \times 10^{14}$ to $20 \times 10^{14}$
negative charges per $cm^2$ of particle area.

The invention will be further described in the following examples, which
are of course not to be construed as limiting the scope of the present
invention.

Example I
Into a 20-litre, three-necked flask equipped with a thermocouple, a
stirrer, a reflux condenser and a dropping funnel there were successively introduced:
an aqueous 5% solution of polymethacrylic acid in water neutralized with
NaOH to a pH of 7,7,
66,8 g of tricalcium phosphate,

7,2 g of sodium chloride,

11520 ml of water, and

17,2 ml of 2N NaOH.

The flask was flushed with nitrogen, followed by stirring (380 revolutions per minute). A solution of 118 g of technical divinyl benzene, (55% by weight) and 3132 g of vinyl toluene (a mixture of 30% para- and 70% ortho-isomers) was added over a period of 30 minutes. To remove residual stabilizers the monomers were washed with a dilute sodium hydroxide solution. Subsequently, 30 g of benzoyl peroxide were added. The stirring speed was increased to 420 revolutions per minute and the reaction mixture heated to 80°C, at which temperature it was kept for 5 hours.

After cooling to room temperature the solid copolymer beads were isolated by filtration. After washing with hydrochloric acid and water they were extracted with petroleum ether, dried to the air and then dried for one hour at 80°C. The beads were screened to obtain 967 g having a diameter of 90 to 150 µm (30%) and 1900 g having a diameter of 150 to 250 µm (58%).


Example II

Into a 2-litre, three-necked flask equipped with a thermometer, a stirrer and a reflux condenser there were introduced 200 g of the copolymer beads of Example I. Subsequently, there were added 500 ml of monochloromethyl ether and 700 ml of trichloroethylene, after which the mixture was allowed to stand for one hour to permit the beads to swell.

The reaction mixture was heated to 60°C with an oil bath, after which a solution of 100 ml of trichloroethylene and 14 g of tintetrachloride was added, followed by stirring for 10 minutes. The reaction mixture was filtered, followed by washing the beads with a 10%-water-containing solution of dioxane until no longer any chloride was detected in the wash liquor. Upon analysis it was found that the beads contained 1,37 meq of chlorine per gramme.


Example III

The procedure of Example II was repeated in such a way that the mixture was only allowed to react for 5 minutes. Upon analysis the beads were found to contain 1,17 meq of chlorine per gramme.

Example IV

The procedure of Example II was repeated in such a way that the reaction time was reduced to 3 minutes. Upon analysis the beads were found to contain 0,8 meq of chlorine per gramme.

Example V

The same procedure was used as in Example II, except that as solvents 400 ml of trichloroethylene and 400 ml of petroleum ether (boiling point 80°-100°C) were used. The reaction time at room temperature was 3 minutes. Upon analysis the beads were found to contain 0,5 meq of chlorine per gramme.

Example VI

Into a 500-ml, three-necked fitted with a thermometer, a stirrer and a reflux condenser there were introduced 20 g of swollen chloromethylated beads (washed with dioxane) of Example VI. Subsequently, 200 ml of diethylamine were added and the contents of the flask were heated on an oil bath (bath temperature 80°C) for 16 hours.

Next, 200 ml of water were slowly added, after which the beads were filtered off and washed with water and alcohol until the wash water was free of amine. The beads were feebly basic and had a capacity of 0,72 meq/g.

Example VII

The same procedure was used as in Example VI, with the exception that there was added a solution of 160 ml of dioxane and 40 ml of trimethylamine. After a reaction time of 16 hours at 35°C strongly basic beads were obtained having a capacity of 0,72 meq per gramme.

Example VIII

The same procedure was used as in Example VII, except that it was started with 50 g of chloromethylated beads having a chlorine content of 2,41 meq per gramme. There were added 400 ml of dioxane and 100 ml of trimethylamine.

The strongly basic beads had a capacity of 2,05 meq per gramme.

Example IX

Into a 500-ml, three-necked fitted with a thermometer, a stirrer and a reflux condenser there were introduced 110 g of copolymer beads of Example I having a diameter of 150 to 250 $\mu$m. There were added 220 ml of sulphuric acid (96%), after which the reaction mixture was subjected to continued stirring for 10 minutes at 23°C.

The beads were successively poured onto ice, removed by filtration and washed with demineralized water until the wash water was free of sulphate. Upon analysis it was found that the beads contained $2,66 \times 10^{-4}$ meq groups of sulphonic acid per gramme, which corresponds to $5 \times 10^{14}$ groups per $cm^2$.

Example X

The procedure of Example IX was repeated in such a way that the mixture was allowed to react for 4 minutes at 40°C. Upon analysis it was found that the beads contained $6,75 \times 10^{-4}$ meq of sulphonic acid groups per gramme, which corresponds to $12,6 \times 10^{4}$ groups per $cm^2$.

Example XI

This example demonstrates that the cell culture microcarriers of the present invention in a number of cell cultures match up to or even excel the well-known cell culture microcarriers based on cross-linked dextran. After washing the cell culture microcarriers with distilled water and flushing with a phosphate buffered salt solution (PBS) a suspension in PBS of 10% by weight of cell culture microcarriers which had stood for 2 hours at room temperature was sterilized in an autoclave for 30 minutes at 120°C. Prior to use the cell culture microcarriers were washed 3 times with the culture medium. Subsequently, about 100 ml of the cell culture microcarriers to be cultivated were introduced into 250 ml spinner bottles equipped with a magnetically driven Teflon-coated stirring bar. The concentration of the cell culture microcarriers in it was 6 $cm^2$/ml. The stirring speed was about 20-60 r.p.m. Cell cultivation was carried out in an appropriate culture medium using cells of the BHK-21-Cl3 type (Baby Hamster Kidney Cells) or human fibroblasts of the $VH_2$ or $SA_7$ type (foreskin fibroblast) or MJB type (human skin fibroblasts, passage 11-17, provided by G.C. Beverstock of the Instituut voor Menselijke Genetica at Leiden). For the growth of the above-mentioned

BHK cells use was made of Eagle's Spinner medium of the RIV (Rijksinstituut voor de Volksgezondheid) supplemented with 10% foetal calf serum (from the firm of Gibco) and a solution containing per litre $3 \times 10^{-7}$ M thymidine $3 \times 10^{-6}$ M hypoxanthine and $10^{-4}$ M non-essential amino acids.

The human fibroblasts were cultivated in MEM (minimum essential medium) supplemented with 10% foetal calf serum and a solution containing per litre $3 \times 10^{-7}$ M thymidine, $3 \times 10^{-6}$ M hypoxanthine and $10^{-4}$ M non-essential amino acids. Cell attachment was determined by taking a 5 ml sample every 2, 4 and 24 hours following inoculation. Then sampling was repeated every 24 hours. The cells attached to the cell culture microcarriers in the samples were counted by using a modification of the method of Sanford et al., as described by Van Wezel in Tissue Culture, Methods and Applications, P.F. Kruse and M.R. Patterson (eds), pp. 372-377 (Academic Press, New York).

The preparation of the cell culture microcarriers was started from the beads with a diameter between 90 and 150 $\mu$m of Example I. After chloromethylation and treatment with trimethylamine cell culture microcarriers were obtained having a capacity of 1,0, 1,4 and 1,8 meq per gramme, respectively. For comparison use was made of dextran beads marketed by Pharmacia Fine Chemicals AB under the trade name Cytodex 1. They had a capacity of 1,5 meq per gramme.

The table below shows the cultivation results expressed as numbers of cells per ml $\times 10^3$, use being made of Baby Hamster Kidney Cells.

Table I

Cells ml $\times 10^3$

| Cell culture microcarriers | Capacity in meq/g | Cultivation periods in hours | | | | |
|---|---|---|---|---|---|---|
| | | 0 | 24 | 48 | 72 | 96 |
| cross-linked p-vinyl toluene | 1,0 | 100 | 170 | 360 | 1700 | 1050 |
| cross-linked p-vinyl toluene | 1,4 | 100 | 220 | 800 | 2150 | 1800 |
| cross-linked p-vinyl toluene | 1,8 | 100 | 230 | 660 | 1850 | 1700 |
| Cytodex 1 | 1,5 | 100 | 180 | 840 | 2100 | 1650 |

The above table clearly shows that the value of 1,4 meq/g for the number of attached BHK cells after 96 hours' cultivation with the cell culture microcarriers of the invention perfectly compares with the number of BHK cells obtained with the well-known dextran beads.

The former, however, have the advantage that they swell to a lesser extent, have a narrow particle size distribution and moreover may be regenerated.

The following table lists the cultivation results expressed as numbers of cells per ml x $10^3$, use being made of $SA_7$ fibroblasts.

## Table II

Cells ml x $10^3$

| Cell culture microcarriers | Capacity in meq/g | Cultivation periods in hours | | | | |
|---|---|---|---|---|---|---|
| | | 0 | 24 | 48 | 72 | 96 |
| cross-linked p-vinyl toluene | 1,0 | 100 | 70 | 120 | 200 | 230 |
| cross-linked p-vinyl toluene | 1,4 | 100 | 110 | 270 | 380 | 570 |
| cross-linked p-vinyl toluene | 1,8 | 100 | 90 | 160 | 270 | 340 |
| Cytodex 1 | 1,5 | 100 | 90 | 160 | 270 | 340 |

The above table clearly shows that a culture of $SA_7$ fibroblasts on cell culture microcarriers according to the invention having a capacity of 1,4 meq/g leads to a good cell yield far more quickly than when present on cell culture microcarriers based on dextran.

Table III gives the cultivation results expressed as numbers of cells per ml x $10^3$, use being made of $VH_2$ fibroblasts.

## Table III

Cells ml x $10^3$

| Cell culture microcarriers | Capacity in meq/g | Cultivation periods in hours | | | | |
|---|---|---|---|---|---|---|
| | | 0 | 24 | 48 | 72 | 96 |
| cross-linked p-vinyl toluene | 1,0 | 100 | 69 | 110 | 230 | 280 |
| cross-linked p-vinyl toluene | 1,4 | 100 | 90 | 210 | 420 | 730 |
| cross-linked p-vinyl toluene | 1,8 | 100 | 60 | 110 | 180 | 370 |
| Cytodex 1 | 1,5 | 100 | 90 | 180 | 310 | 390 |

Example XII

In the table below the results of growing BHK-21 cells and MJB fibroblasts on cell culture microcarriers of the invention negatively charged with respectively $6,0 \times 10^{14}$ and $15,3 \times 10^{14}$ sulphonic acid groups per $cm^2$ are compared with the results obtained with a cell culture microcarrier based on polystyrene negatively charged with $2\text{-}10 \times 10^{14}$ sulphonic acid groups per $cm^2$ and with Cytodex positively charged with 1,5 meq of sulphonic acid groups per g.

## Table IV

| Cell culture microcarriers based on | sulphonic acid groeps/$cm^2$ | cells per ml $x10^3$ | | | | | | | | |
|---|---|---|---|---|---|---|---|---|---|---|
| | | Cultivation periods in hours | | | | | | | | |
| | | 0 | 4 | 24 | 48 | 72 | 120 | 144 | 192 | 240 |
| p-vinyl toluene | $6x10^{14}/cm^2$ | 100 | 100 | 120 | 120 | 130 | 150 | 160 | 210 | 200 |
| p-vinyl toluene | $15,3x10^{14}/cm^2$ | 100 | 80 | 120 | 140 | 180 | 220 | 250 | 250 | 340 |
| polystyrene | $2-10x10^{14}/cm^2$ | 100 | 100 | 110 | - | 90 | 90 | - | 80 | 90 |
| dextran capacity (positive) in meq/g | 1,5 meq/g | 100 | 70 | 85 | 95 | 90 | 90 | 90 | 130 | 170 |

The above table clearly shows that the cell culture microcarriers of the invention based on p-vinyl toluene lead to far better cultivation results than the well-known cell culture microcarriers based on polystyrene or dextran.

Also the growth of BHK-21 cells on cell culture microcarriers based on p-vinyl toluene was found to proceed far more quickly than on cell culture microcarriers based on polystyrene or dextran.

The cultivation results obtained with the same cell culture microcarriers as mentioned in Table IV using BHK-21 cells are given in Table V.

## Table V

| Cell culture microcarriers based on | sulphonic acid groeps/cm$^2$ | cells per ml x10$^3$ | | | | |
|---|---|---|---|---|---|---|
| | | Cultivation periods in hours | | | | |
| | | 0 | 4 | 24 | 48 | 72 |
| p-vinyl toluene | 6x10$^{14}$/cm$^2$ | 100 | 130 | 480 | 1340 | 1740 |
| p-vinyl toluene | 15,3x10$^{14}$/cm$^2$ | 100 | 90 | 430 | 1700 | 1840 |
| polystyrene | 2-10x10$^{14}$/cm$^2$ | 100 | 110 | 260 | 850 | 1030 |
| dextran capacity (positive) in meq/g | 1,5 meq/g | 100 | 110 | 180 | 950 | 1630 |

CLAIMS

1.  Cell culture microcarriers having a diameter in the range of from 60 to 300 μm, the carrier material consisting of the polymerization product of an aromatic monovinyl hydrocarbon provided with negatively or positively charged groups, characterized in that the polymerization product substantially consists of cross-linked polyvinyl toluene.

2.  Cell culture microcarriers according to claim 1, characterized in that the cross-linking agent is used in an amount of 0,1 to 7% by weight, calculated on the polyvinyl toluene.

3.  Cell culture microcarriers according to claim 2, characterized in that the amount of cross-linking agent is 0,1 to 4% by weight, preferably 0,5-2,5% by weight.

4.  Cell culture microcarriers according to claim 1, characterized in that the cross-linking agent is divinyl benzene.

5.  A process of growing anchorage-dependent cells in microcarrier culture, characterized in that use is made of cell culture microcarriers according to one or more of the preceding claims.